# FASCICULE DE BREVET EUROPEEN

(11) **EP 2 510 974 B1**
(45) Date de publication et mention de la délivrance du brevet: **25.12.2013**
(21) Numéro de dépôt: 12153727.8
(22) Date de dépôt: 02.02.2012
(51) Int. Cl.: A61N 1/37, A61B 5/0456, G06F 17/00

(54) **Dispositif médical actif, notamment défibrillateur implantable, à détection des complexes QRS dans un signal fortement bruité**
Aktive medizinische Vorrichtung, insbesondere implantierbarer Defibrillator, zur Erkennung von QRS-Komplexen in einem stark verrauschten Signal
Active medical device, in particular an implantable defibrillator, with detection of QRS complexes in a signal with a lot of interference

(30) Priorité: 15.04.2011 FR 1153301
(43) Date de publication de la demande: 17.10.2012
(73) Titulaire: Sorin CRM SAS, 92140 Clamart (FR)
(72) Inventeur: Bonan, José, 75014 Paris (FR); Harmel, Christophe, 92330 Sceaux (FR)
(74) Mandataire: Dupuis-Latour, Dominique

(56) Documents cités:
- EP-A1- 0 958 843
- EP-A2- 0 429 025
- US-A- 4 000 461
- US-A- 6 161 037

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif. L'analyse du rythme cardiaque est effectuée à partir de signaux d'électrogramme (EGM) recueillis par des électrodes portées par des sondes endocavitaires implantées dans le myocarde pour mesurer le potentiel de dépolarisation auriculaire et/ou ventriculaire. Ces signaux sont analysés par l'implant, qui délivre si besoin au patient une thérapie appropriée sous forme d'impulsions de faible énergie (stimulation antibradycardique ou stimulation de resynchronisation des ventricules), ou de chocs de défibrillation.

L'analyse du rythme, et donc la décision de délivrer ou non une thérapie, peut être cependant perturbée par des artefacts recueillis par la sonde endocavitaire.

Ces artefacts peuvent être d'origines diverses. Une première série d'artefacts correspond à des situations où le dispositif détecte non seulement l'évènement proprement dit, c'est-à-dire l'onde de dépolarisation de la cavité considérée, mais également une perturbation associée à ce même événement et considérée, à tort, comme un autre événement survenu après le premier : onde de dépolarisation tardive, diaphonie entre cavités, *far-field,* etc.

Une autre série d'artefacts, qui sont ceux concernés par la présente invention, sont les artefacts de bruit extrinsèque, non lié à la dépolarisation du myocarde. Ce bruit peut avoir plusieurs origines, dont notamment les myopotentiels associés aux contractions musculaires, ainsi que les interférences électromagnétiques (EMI) provenant d'équipements électroniques de surveillance, d'appareils électriques environnants, d'instruments électrochirurgicaux, de systèmes de communication, etc.

Au surplus, le traitement numérique du signal engendre par lui-même un certain niveau de bruit, qui se surajoute au signal de dépolarisation. Notamment, les filtrages numériques ajoutent au signal des oscillations dont l'amplitude diminue lentement avant de se stabiliser, ce qui a pour conséquence de perturber l'analyse opérée en aval de ce signal.

De façon générale, un bruit présent avec plus ou moins de régularité, qu'il soit extrinsèque ou propre au traitement numérique, peut être interprété par l'implant comme une dépolarisation du myocarde, avec le risque de générer des thérapies inappropriées, par exemple en inhibant à tort les stimulations antibradycardiques ou de resynchronisation ou, inversement, en délivrant à tort des chocs inappropriés.

Diverses techniques ont été proposées pour réduire l'incidence des bruits extrinsèques, notamment l'application en amont de la chaîne de traitement d'un filtrage analogique ou numérique, l'instauration de périodes réfractaires, l'adaptation automatique de la sensibilité des amplificateurs de détection, ou le contrôle automatique du gain de ces amplificateurs.

Le EP 0 958 843 A1 décrit une telle technique d'*autosensing,* où un algorithme adapte en permanence le seuil de détection en fonction du niveau du bruit et de l'amplitude des signaux EGM associés à des événements détectés.

Cependant, l'utilisation de ces divers moyens se fait toujours au détriment d'une bonne détection.

En particulier, pour détecter la fibrillation ventriculaire (FV), dont le niveau du signal est faible, il est nécessaire de disposer d'une sensibilité maximale, sous peine de ne pas détecter des événements qui auraient dû l'être.

Mais l'amplitude des signaux de fibrillation ventriculaire (les complexes QRS, représentatifs des dépolarisations du ventricule) peut se situer à un niveau variable, intermédiaire entre le niveau du bruit et celui des signaux des complexes sinusaux.

Si l'on veut pouvoir détecter la fibrillation ventriculaire, la détection d'un bruit éventuel est donc inévitable.

Si de plus on se trouve en présence d'un bruit régulier, pour un patient présentant un rythme cardiaque sinusal normal ce bruit peut être confondu avec des dépolarisations. Cette situation peut fausser l'évaluation du rythme moyen par l'implant, avec un rythme estimé (à tort) à un niveau très supérieur à la réalité, et un risque corrélatif d'application d'une thérapie antitachycardique indésirable (situation de faux positif, dite *oversensing).*

Inversement, si l'appareil est programmé à une valeur de sensibilité trop faible, c'est-à-dire avec un seuil de détection trop élevé, les épisodes réels de fibrillation ventriculaire risquent de ne pas être détectés (situation de faux négatif), avec pour le patient des conséquences qui peuvent être très graves.

La détection de bruits extrinsèques étant généralement inévitable, le problème de l'invention consiste à discriminer ces bruits parmi les dépolarisations cardiaques, ceci afin d'éviter le déclenchement de traitements inappropriés ou, inversement, l'inhibition de traitements qui auraient été justifiés.

L'identification de ces bruits et leur atténuation au moyen de filtres est une tâche complexe, la principale difficulté résidant dans l'extrême variabilité de ces composantes parasites, ce qui implique des traitements relativement complexes si l'on veut qu'ils soient efficaces.

Des techniques complexes de traitement numérique ont été proposées à cet effet pour assurer la détection d'un complexe QRS dans le signal endocavitaire : filtrage non linéaire, transformation par ondelettes, réseau artificiel, algorithme génétique, prédiction linéaire, etc. Ces algorithmes divers, complexes, nécessitent un nombre important d'opérations arithmétiques et nécessitent des ressources de calcul importantes avec en outre, une augmentation importante de la consommation du dispositif, donc avec une incidence sur sa durée de vie finale.

Une autre technique, décrite par exemple dans le EP 0 775 502 (ELA Medical), consiste à analyser les caractéristiques du signal en dérivant celui-ci par des moyens différentiateurs, pour évaluer les variations instantanées de ce signal en les comparant à des seuils prédéterminés. Les circuits nécessaires sont relativement simples mais en revanche, la discrimination qu'ils permettent est relativement limitée dans le cas de signaux fortement bruités.

Une autre technique encore a été proposée par le EP 1 857 142 A1 (ELA Medical), qui consiste à procéder à une double détection : de la dépolarisation par analyse du signal électrique EGM, et de la contraction du myocarde par mesure de l'accélération endocardiaque, via un accéléromètre directement en contact avec le muscle cardiaque. En présence de bruits suspects, le dispositif opère une levée de doute pour confirmer que le signal détecté a été effectivement suivi par une activité mécanique du coeur et constitue donc bien un signal de dépolarisation (un complexe QRS) et non un artefact : la dépolarisation, qui est un phénomène électrique sensible au bruit, est en effet normalement suivie d'une contraction cardiaque, qui est un phénomène mécanique qui n'est pas affecté par le bruit. Mais ceci présuppose de disposer d'une sonde équipée d'un capteur d'accélération endocardiaque et, si le patient est déjà appareillé, implique lors d'un changement de générateur de changer non seulement le générateur mais également la sonde, ce qui est beaucoup plus délicat sur le plan chirurgical.

Le but de l'invention est de proposer un traitement du signal endocavitaire permettant de discriminer de façon efficace les composantes parasites de bruit, de manière à détecter de façon fiable la présence (ou non) d'un complexe QRS dans le signal endocavitaire recueilli.

Le but de l'invention est de permettre un tel traitement avec une économie maximale des moyens matériels, notamment des besoins réduits en puissance de calcul pour le traitement numérique, avec pour conséquence une préservation de l'énergie de la batterie du dispositif et donc de sa durée de vie.

Le but de l'invention est également de proposer un tel traitement qui soit utilisable indépendamment du type de sonde utilisée, cette caractéristique étant avantageuse notamment lorsqu'il s'agit de remplacer seulement le générateur du dispositif, sans remplacement de la sonde déjà présente. Le EP 0 429 025 A2 expose une technique consistant à opérer une double discrimination, à la fois sur l'amplitude et sur la largeur des pics de signal détectés : une première discrimination sur l'amplitude permet d'extraire les seuls signaux dépassant un niveau donné, tandis que l'analyse de la largeur du signal dépassant ce seuil d'amplitude permet de discriminer une éventuelle composante parasite qui n'aurait pas été éliminée au filtrage en amont de ce traitement. Le US 4 000 461 A expose une technique comparable de discrimination par analyse croisée amplitude/durée des pics de signal détectés.

Les seuils de comparaison (en amplitude et en largeur) sont paramétrables, et ajustés à une valeur choisie par le praticien lors de la programmation de l'implant.

Ceci présuppose toutefois que les conditions qui prévalaient au moment du réglage restent à peu près constantes au cours du temps, si l'on souhaite une discrimination efficace.

Mais en pratique cela est rarement le cas, ce qui rend cette technique peu fiable pour détecter la présence (ou non) d'un complexe QRS dans le signal endocavitaire recueilli, tout particulièrement pour détecter une fibrillation ventriculaire pour laquelle, comme on l'a expliqué ci-dessus, le signal utile peut se situer à un niveau variable, intermédiaire entre le niveau du bruit et celui des signaux des complexes sinusaux. Du fait du paramétrage figé, les risques d'*oversensing* ou d'*undersensing* sont alors particulièrement élevés, avec les graves conséquences que l'on a exposées plus haut.

Tel est le problème de l'invention.

La solution de l'invention consiste, essentiellement, à prévoir une discrimination à double seuil (amplitude et durée du pic), mais où le seuil d'amplitude pour la discrimination entre signal utile et bruit est un seuil adaptatif, variable, mis à jour en fonction du niveau moyen de bruit déterminé en l'absence de signal détecté. Le seuil d'amplitude de discrimination sera ainsi mis à jour régulièrement en fonction du niveau de dépassement du seuil.

Plus précisément, l'invention propose un dispositif du type générique divulgué par le EP 0 429 025 A2 précité, c'est-à-dire comprenant des moyens de recueil, d'amplification, de préfiltrage et de conversion analogique/numérique d'un signal endocavitaire, et des moyens numériques de traitement et d'analyse du signal endocavitaire numérisé, aptes à délivrer un indicateur de détection de pic de signal, représentatif de la présence d'un complexe QRS dans le signal endocavitaire. Ces moyens numériques de traitement et d'analyse comportent un comparateur à double seuil, apte à recevoir en entrée le signal numérisé et à délivrer en sortie l'indicateur de détection de pic lorsque, cumulativement : l'amplitude du signal en entrée dépasse un seuil d'amplitude de pic, et ce seuil d'amplitude de pic est dépassé pendant une durée supérieure à un seuil de largeur de pic.

De façon caractéristique de l'invention, le seuil d'amplitude de pic est un seuil adaptatif variable, et les moyens numériques de traitement et d'analyse comportent en outre des moyens de détermination du seuil d'amplitude de pic, aptes à combiner une valeur prédéterminée de seuil de base à une valeur variable de niveau de bruit du signal en entrée, et des moyens de détermination de ladite valeur variable de niveau de bruit à partir de l'énergie du bruit présent dans le signal en entrée hors des périodes de détection de pic de signal.

En particulier, les moyens de détermination de la valeur variable de niveau de bruit peuvent être des moyens aptes à calculer l'énergie du signal en entrée sur une durée de calcul prédéterminée pendant laquelle l'amplitude du signal en entrée ne dépasse pas ledit seuil d'amplitude de pic.

Le dispositif peut notamment comprendre en outre des moyens pour inhiber et réinitialiser les moyens de détermination de la valeur variable de niveau de bruit sur franchissement dudit seuil d'amplitude de pic avant expiration de ladite durée de calcul prédéterminée.

Le seuil de largeur de pic est de préférence un seuil prédéterminé fixe.

Avantageusement, les moyens numériques de traitement et d'analyse comportent en outre un filtre passe-bas apte à filtrer le signal en entrée, ainsi qu'un filtre passe-haut et un redresseur, aptes à filtrer et redresser le signal appliqué au filtre passe-bas.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 présente, sous forme de schéma synoptique par blocs, l'ensemble du système de détection des complexes QRS selon l'invention.
La Figure 2 illustre la manière dont est opérée la double détection, en amplitude et en largeur.
La Figure 3 est un diagramme de séquencement de type machine à états des différents états de l'algorithme d'analyse et de mise à jour du seuil adaptatif selon l'invention.
Les Figures 4a à 4d montrent diverses configurations possibles de l'onde de dépolarisation, pour illustrer la manière dont sont opérées la détection du complexe QRS et la mise à jour du seuil d'amplitude.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Par ailleurs, on notera que le procédé de l'invention est mis en oeuvre par des moyens principalement logiciels, avec des algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

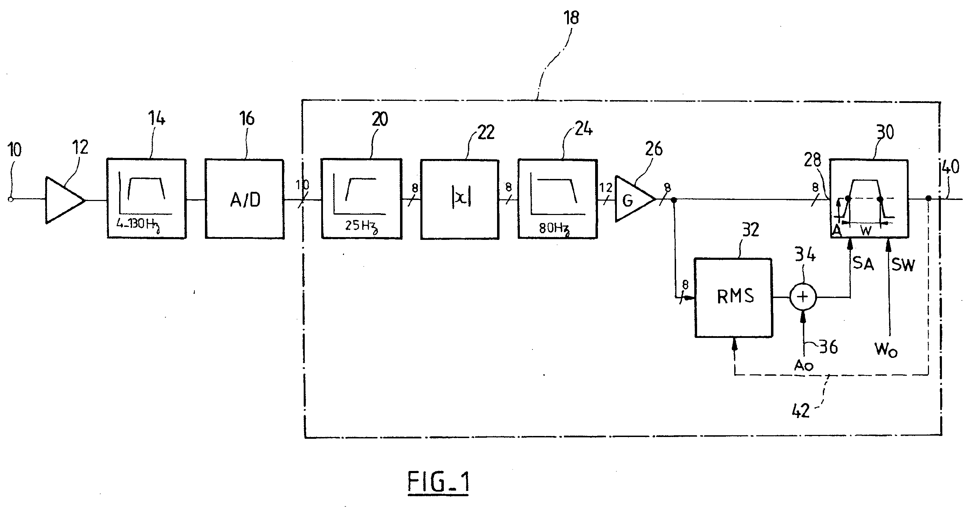

La Figure 1 illustre, sous forme de blocs fonctionnels, les différents éléments permettant la détection du complexe QRS d'un signal de dépolarisation ventriculaire, notamment en cas de fibrillation ventriculaire, à partir d'un signal endocavitaire recueilli par l'électrode située à l'extrémité d'une sonde reliée au générateur incorporant cette chaîne de détection.

Ce système comprend une partie analogique suivie d'une partie numérique.

Tout d'abord, le signal EGM endocavitaire est appliqué sur l'entrée 10 d'un amplificateur 12 et d'un filtre passe-bande analogique 14, par exemple un filtre de type Butterworth, défini dans une limite relativement large (typiquement entre 4 et 130 Hz).

Le signal amplifié et filtré fait ensuite l'objet d'une conversion analogique/numérique par un convertisseur 16, par exemple une conversion sur 10 bits à une fréquence d'échantillonnage de 512 Hz.

Le signal brut numérisé est traité par le bloc numérique 18, afin d'y détecter de la présence d'un complexe QRS.

Le signal numérisé issu du convertisseur 16 est appliqué à un filtre passe-haut 20 de manière à supprimer la composante continue éventuellement présente (et ainsi éviter une saturation des circuits de traitement en aval), et également pour renforcer certaines composantes caractéristiques du signal EGM. Ce filtre passe-haut 20 est par exemple un filtre Butterworth du troisième ordre avec une fréquence de coupure de 25 Hz . Un tel filtre procure une stabilisation rapide du signal en sortie, avec peu d'oscillations (pour un signal échelon appliqué en entrée). Il présente en outre l'avantage de ne consommer qu'une faible puissance et être peu exigeant en termes de ressources de calcul.

L'étage suivant (bloc 22) est un étage redresseur, qui rend positifs tous les points du signal numérisé et permet ainsi de simplifier les opérations arithmétiques ultérieures. Cette fonction introduit certes des non-linéarités, mais simplifie les traitements ultérieurs (seuil positif uniquement lorsqu'il s'agit d'évaluer l'amplitude du signal), et assure une plus grande flexibilité et une simplification des règles de décision de la présence ou non d'un complexe QRS.

L'étage redresseur 22 est suivi par un filtre passe-bas (bloc 24), dont le but est d'augmenter la résolution en largeur du signal redressé et filtré, car comme on le verra plus loin l'analyse de ce signal implique une analyse en largeur des pics de signal qui ont été détectés, pour accroître l'efficacité de la discrimination par rapport au bruit.

Ce filtre passe-bas 24 est par exemple un filtre Butterworth du deuxième ordre avec une fréquence de coupure de l'ordre de 80 Hz, et de préférence un filtre numérique à réponse impulsionnelle infinie (IIR), qui présente un faible temps de réponse. Un filtre rapide offre en effet la possibilité de démarrer plus rapidement l'algorithme de détection du complexe QRS.

Le signal ainsi filtré en sortie du bloc 24 est appliqué en entrée d'un étage de gain 26, dont la valeur est choisie de manière à ne pas saturer le système en aval.

Le signal résultant est appliqué à l'entrée 28 d'un comparateur 30, qui est un comparateur à double seuil : d'amplitude et de largeur.

Ce comparateur 30 analyse tout d'abord l'amplitude A du signal par rapport au seuil d'amplitude SA puis, lorsque ce seuil d'amplitude est franchi, analyse la largeur W du pic d'amplitude par rapport au seuil de largeur SW. En d'autres termes, pour que le comparateur bascule il est nécessaire à la fois que l'amplitude A du signal dépasse le seuil SA, et que ce seuil SA soit dépassé pendant une durée W (largeur du pic) supérieure au seuil de largeur SW.

Le seuil de largeur SW est une valeur prédéterminée W₀, fixe ou paramétrée.

En revanche, le seuil d'amplitude SA est un seuil variable, adaptatif en fonction du niveau de bruit moyen à une période donnée.

Ce seuil d'amplitude adaptatif SA est déterminé au moyen d'un circuit 32 de détermination du niveau de bruit. Le circuit 32 reçoit en entrée le même signal que celui appliqué à l'entrée du comparateur 30, mais seulement hors des périodes de dépassement du seuil d'amplitude. Le niveau de bruit est par exemple déterminé par l'énergie, c'est-à-dire la valeur efficace (racine carrée de la moyenne des carrés ou RMS) d'une série d'échantillons pendant une durée prédéterminée T_{RMS}. Pour des raisons d'économie d'énergie, ce calcul de valeur efficace RMS est limité à une durée relativement courte, par exemple T_{RMS} = 32 ms.

La valeur moyenne obtenue RMS est ajoutée par un additionneur 34 à une valeur prédéterminée de seuil de base A₀ appliquée également en entrée 36 de l'additionneur 34. La valeur résultante SA = A₀ + RMS est appliquée en entrée du comparateur 30 en tant que valeur de référence du seuil d'amplitude SA.

Le circuit 32 de calcul de la valeur efficace RMS est, comme indiqué par la ligne 42, couplé à la sortie 40 du comparateur 30 de manière à n'opérer le calcul de la valeur efficace que dans certaines conditions de signal, comme on va le décrire plus bas.

En résumé, l'invention propose, après conditionnement préalable du signal numérisé par un filtrage approprié, d'opérer sur le signal d'entrée un tri en termes d'énergie entre les pics de bruit et ceux du signal utile.

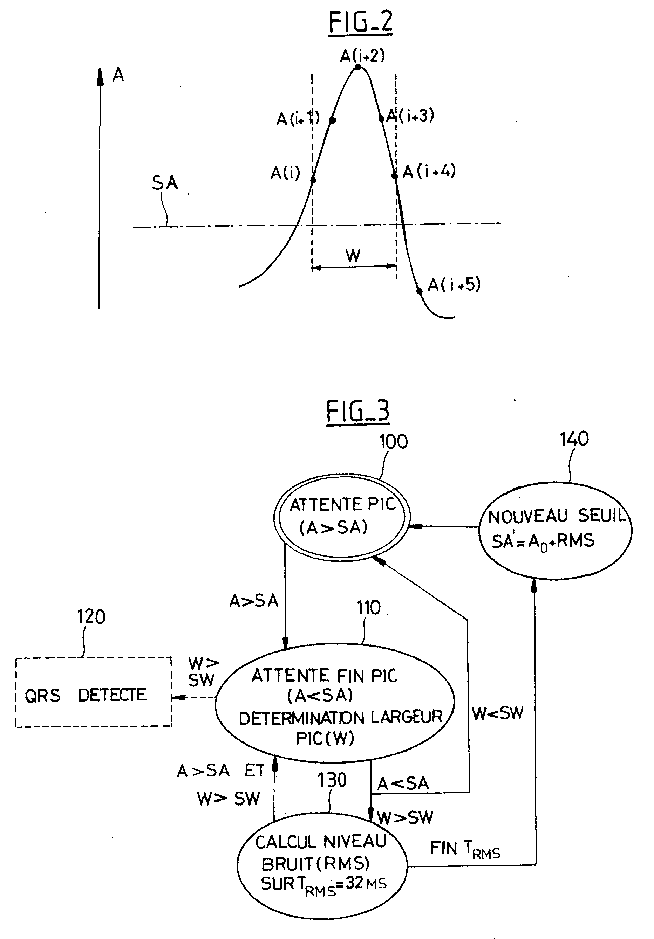

En d'autre termes, la détection d'un pic à l'aide d'un comparateur à double seuil (amplitude et largeur) offre la possibilité de décorréler le signal utile d'avec le bruit et détermine, sous réserve de certaines conditions (voir notamment ci-dessous les explications données en référence à la Figure 3), le déclenchement du calcul de la valeur moyenne RMS du bruit associé au signal. Cette valeur, calculée sur un certain laps de temps combiné à une valeur de seuil prédéterminée, permet une mise à jour régulière du seuil en amplitude.

Sur la Figure 2, on a illustré une forme de signal numérisé filtré tel qu'appliqué en entrée du comparateur à double seuil 30, signal qui se présente sous forme d'une succession d'échantillons discrets Aᵢ, Aᵢ₊₁,...

Le comparateur détecte d'abord les échantillons dont l'amplitude A dépasse le seuil d'amplitude SA. Si la largeur W du pic de signal (c'est-à-dire la partie du signal qui dépasse le seuil SA) dépasse un seuil de largeur SW (c'est-à-dire si le nombre d'échantillons consécutifs situés au-dessus du seuil SA dépasse un nombre donné), alors le pic de signal est considéré comme représentant effectivement un complexe QRS. En d'autre termes, si les deux seuils d'amplitude et de largeur sont cumulativement franchis, le dispositif considère qu'il y a présence effective d'une dépolarisation ventriculaire.

Comme on l'a indiqué plus haut, le seuil de largeur SW est un seuil fixe Wo, paramétrable, tandis que le seuil d'amplitude SA est un seuil variable, adaptatif, qui varie avec le niveau de bruit du signal analysé, ce bruit étant constitué par toutes les fluctuations aléatoires non représentatives de la dépolarisation cardiaque.

La Figure 3 illustre sous forme d'un diagramme de type machine à états l'enchaînement des différentes étapes de l'algorithme pour i) la détection du QRS, ii) le calcul du niveau de bruit et iii) la mise à jour du seuil d'amplitude.

Initialement (bloc 100), le dispositif est en attente d'un pic, c'est-à-dire en attente d'un échantillon dont l'amplitude A dépasse le seuil d'amplitude SA (A > SA).

Si un tel pic est détecté, l'étape suivante (bloc 110) consiste à attendre la fin du pic (c'est-à-dire à détecter le premier échantillon suivant dont l'amplitude A retombe au-dessous du niveau de seuil d'amplitude SA). La largeur W du pic est alors déterminée (cette largeur correspondant au nombre d'échantillons consécutifs situés au-dessus du seuil d'amplitude SA). Si cette largeur W est supérieure au seuil de largeur SW, alors on considère qu'un complexe QRS a été effectivement détecté (bloc 120). Dans la négative, l'algorithme retourne au bloc 100.

Par ailleurs, dès que l'amplitude A retombe au-dessous du seuil SA (A < SA), l'algorithme déclenche le calcul du niveau de bruit (bloc 130). Ce calcul est opéré sur une durée donnée T_{RMS} (par exemple T_{RMS} = 32 ms), et à condition que l'amplitude reste au-dessous du seuil SA ; dans le cas contraire, l'algorithme annule le calcul du niveau de bruit et retourne au bloc 110 (en d'autres termes, un nouveau pic vient d'être détecté et va être analysé, et le temps imparti T_{RMS} n'a pas été suffisant pour le calcul du bruit).

Si la période T_{RMS} a pu s'écouler entièrement avec l'amplitude restant au-dessous du seuil SA, alors ce seuil est mis à jour (bloc 140). Le nouveau seuil SA' est calculé avec la nouvelle valeur mise à jour du bruit RMS (SA' = SA + RMS).

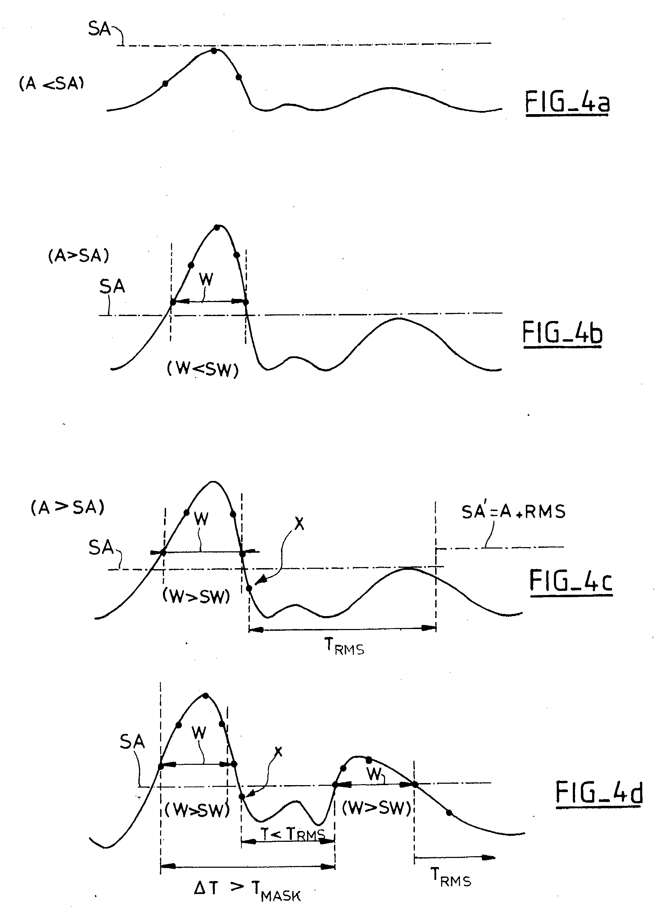

Les Figures 4a à 4d illustrent quatre configurations différentes du signal analysé appliqué en entrée du comparateur à double seuil, pour illustrer les actions correspondantes prises selon le cas.

La Figure 4a illustre un signal dont l'amplitude A reste en permanence au-dessous du seuil SA : dans ce cas, la sortie du comparateur à double seuil 30 reste à '0' ("absence de QRS détecté"), et la valeur du seuil SA reste inchangée. Aucun calcul de niveau de bruit n'est déclenché.

La Figure 4b illustre un signal dont l'amplitude A dépasse le seuil SA, mais sur une durée (largeur W) inférieure au seuil de largeur SW prescrit. Dans ce cas, le pic détecté est vraisemblablement un pic parasite de bruit, et aucune action n'est prise, comme dans le cas précédent.

La Figure 4c illustre le cas où le signal dépasse le seuil d'amplitude SA pendant une durée W supérieure au seuil de largeur SW. Dans ce cas, on considère qu'il y a détection d'un QRS (sortie '1' délivrée par le comparateur 30). On notera que ce signal de détection est délivré avec un retard par rapport au début du pic, retard égal au nombre d'échantillons nécessaires pour atteindre le seuil de largeur SW. Mais ce retard est relativement faible : dès que le seuil SW est franchi, le signal est délivré par le comparateur.

Une fois le seuil de largeur SW atteint, le système attend que l'amplitude retombe au-dessous du seuil SA (échantillon désigné X sur la Figure 4c). Le calcul du niveau de bruit est alors déclenché, pendant la durée T_{RMS} prescrite (T_{RMS} = 32 ms). Pendant toute la durée T_{RMS} du calcul du niveau de bruit, la valeur de seuil d'amplitude SA est maintenue à sa valeur antérieure. À la fin du calcul, le seuil d'amplitude est automatiquement actualisé, de la valeur SA à la valeur SA' = A₀ + RMS (A₀ étant le seuil de base et RMS étant la valeur du niveau de bruit qui vient juste d'être calculé). Enfin, la Figure 4d illustre une configuration dans laquelle deux pics rapprochés sont successivement détectés, l'instant de début du second pic étant situé à un intervalle de temps ΔT du début du premier pic. Dans ce cas :
- si ΔT est inférieur à une durée prédéterminée T_{MASK}, les deux pics successifs sont considérés comme un pic unique, et un seul signal de détection de QRS est délivré en sortie (les deux pics rapprochés sont assimilés à un complexe QRS unique) ;
- si en revanche le second pic est détecté avec ΔT > T_{MASK}, alors les deux pics sont considérés comme deux pics séparés, représentatifs de deux complexes QRS distincts ;
- d'autre part, si le début du second pic est détecté avant la fin de la période T_{RMS} de calcul du niveau de bruit, alors ce calcul est annulé et le seuil d'amplitude SA est maintenu inchangé. Le calcul du niveau de bruit sera repris ultérieurement, après la fin du second pic d'amplitude.

La technique de détection selon l'invention a fait l'objet de tests sur une vingtaine de dispositifs implantés.

Avec les systèmes de détection conventionnels, le taux moyen de détection de faux positifs (*oversensing*), c'est-à-dire le ratio du nombre de pics d'amplitude détectés à tort comme des QRS au nombre total d'indicateurs de détection de pic délivrés en sortie, était de l'ordre de 80 %. Avec l'invention, ce taux moyen a pu être réduit à environ 18 %, soit une diminution de plus de quatre fois du nombre de fausses détections, toutes choses égales par ailleurs.

## Revendications

1. Un dispositif médical implantable actif, comprenant des moyens (12-16) de recueil, d'amplification, de préfiltrage et de conversion analogique/numérique d'un signal endocavitaire, et des moyens numériques (18) de traitement et d'analyse du signal endocavitaire numérisé, aptes à délivrer un indicateur de détection de pic de signal, représentatif de la présence d'un complexe QRS dans le signal endocavitaire,
dans lequel les moyens numériques de traitement et d'analyse comportent un comparateur à double seuil (30), apte à recevoir en entrée (28) le signal numérisé et à délivrer en sortie (40) ledit indicateur de détection de pic lorsque, cumulativement :
· l'amplitude (A) du signal en entrée dépasse un seuil d'amplitude de pic (SA), et
· ledit seuil d'amplitude de pic est dépassé pendant une durée (W) supérieure à un seuil de largeur de pic (SW),
**caractérisé en ce que** ledit seuil d'amplitude de pic (SA) est un seuil adaptatif variable, et **en ce que** les moyens numériques de traitement et d'analyse comportent en outre :
- des moyens (34) de détermination dudit seuil d'amplitude de pic (SA), aptes à combiner une valeur prédéterminée de seuil de base (A₀) à une valeur variable de niveau de bruit du signal en entrée ; et
- des moyens (32) de détermination de ladite valeur variable de niveau de bruit à partir de l'énergie (RMS) du bruit présent dans le signal en entrée hors des périodes de détection de pic de signal.

2. Le dispositif de la revendication 1, dans lequel les moyens (32) de détermination de la valeur variable de niveau de bruit sont des moyens aptes à calculer l'énergie du signal en entrée sur une durée de calcul prédéterminée (T_{RMS}) pendant laquelle l'amplitude du signal en entrée ne dépasse pas ledit seuil d'amplitude de pic (SA).

3. Le dispositif de la revendication 2, comprenant en outre des moyens (32, 42) pour inhiber et réinitialiser les moyens de détermination de la valeur variable de niveau de bruit sur franchissement dudit seuil d'amplitude de pic avant expiration de ladite durée de calcul prédéterminée.

4. Le dispositif de la revendication 1, dans lequel ledit seuil de largeur de pic (SW) est un seuil prédéterminé fixe (Wo).

5. Le dispositif de la revendication 1, dans lequel les moyens numériques de traitement et d'analyse comportent en outre :
- un filtre passe-bas (24), apte à filtrer ledit signal en entrée.

6. Le dispositif de la revendication 5, dans lequel les moyens numériques de traitement et d'analyse comportent en outre :
- un filtre passe-haut (20) et un redresseur (22), aptes à filtrer et redresser le signal appliqué au filtre passe-bas.

## Patentansprüche

1. Aktive implantierbare medizinische Vorrichtung, umfassend Mittel (12 bis 16) zum Abfangen, Verstärken, Vorfiltern und Analog-/Digital-Wandeln eines endokavitären Signals und digitale Mittel (18) zum Verarbeiten und Analysieren des digitalisierten endokavitären Signals, die dazu geeignet sind, um einen Signalspitzen-Detektionsindikator abzugeben, der das Vorliegen eines QRS-Komplexes in dem endokavitären Signal darstellt,
wobei die digitalen Mittel zur Verarbeitung und Analyse einen Dual-Komparator (30) umfassen, der dazu geeignet ist, um am Eingang (28) das digitalisierte Signal zu empfangen und am Ausgang (40) den Spitzen-Detektionsindikator abzugeben, wenn kumulativ:
• die Amplitude (A) des Eingangssignals eine Spitzenamplitudenschwelle (SA) übersteigt, und
• die Spitzenamplitudenschwelle während einer Dauer (W) überschritten wird, die größer als eine Spitzenbreitenschwelle (SW) ist,
**dadurch gekennzeichnet, dass** die Spitzenamplitudenschwelle (SA) eine variable adaptive Schwelle ist, und dass die digitalen Mittel zur Verarbeitung und Analyse ferner Folgendes umfassen:
- Mittel (34) zum Bestimmen der Schwellenamplitudenspitze (SA), die dazu geeignet sind, um einen vorbestimmten Basisschwellenwert (A₀) mit einem variablen Rauschpegelwert des Eingangssignals zu kombinieren; und
- Mittel (32) zum Bestimmen des variablen Rauschpegelwertes aus der Energie (RMS) des Rauschens, das in dem Eingangssignal außerhalb der Zeiträume zum Erkennen der Signalspitze vorliegt.

2. Vorrichtung nach Anspruch 1, wobei die Mittel (32) zum Bestimmen des variablen Rauschpegelwertes Mittel sind, die in der Lage sind, die Energie des Eingangssignals über eine vorbestimmte Berechnungsdauer (T_{RMS}) zu berechnen, während der die Amplitude des Eingangssignals die Spitzenamplitudenschwelle (SA) nicht übersteigt.

3. Vorrichtung nach Anspruch 2, ferner umfassend Mittel (32, 42) zum Sperren und Rückstellen der Mittel zum Bestimmen des variablen Rauschpegelwertes beim Überschreiten der Spitzenamplitudenschwelle vor Ablauf der vorbestimmten Berechnungsdauer.

4. Vorrichtung nach Anspruch 1, wobei die Spitzenbreitenschwelle (SW) eine feste vorbestimmte Schwelle (Wo) ist.

5. Vorrichtung nach Anspruch 1, wobei die digitalen Mittel zur Verarbeitung und Analyse ferner Folgendes umfassen:
- ein Tiefpassfilter (24), das geeignet ist, um das Eingangssignal zu filtern.

6. Vorrichtung nach Anspruch 5, wobei die digitalen Mittel zur Verarbeitung und Analyse ferner Folgendes umfassen:
- ein Hochpassfilter (20) und einen Gleichrichter (22), die dazu geeignet sind, um das Signal, das auf das Tiefpassfilter angewendet wird, zu filtern und gleichzurichten.

## Claims

1. An active implantable medical device, comprising means (12-16) for collecting, amplifying, prefiltering and analog-to-digital converting an endocavitary signal, and digital means (18) for processing and analysing the digitized endocavitary signal, adapted to deliver an indicator of detection of signal peak, representative of the presence of a QRS complex in the endocavitary signal,
wherein the digital processing and analysis means include a double-threshold comparator (30), adapted to receive at the input (28) the digitized signal and to deliver at the output (40) said peak detection indicator when, cumulatively:
- the amplitude (A) of the input signal exceeds a peak amplitude threshold (SA), and
- said peak amplitude threshold is exceeded for a duration (W) longer than a peak width threshold (SW), **characterized in that** said peak amplitude threshold (SA) is a variable adaptive threshold, and **in that** the digital processing and analysis means further include:
- means (34) for determining said peak amplitude threshold (SA), adapted to combine a predetermined base threshold value (A₀) to a variable noise level value of the input signal; and
- means (32) for determining said variable noise level value based on the energy (RMS) of the noise present in the input signal, out of the periods of signal peak detection.

2. The device of claim 1, wherein the means (32) for determining the variable noise level value are means adapted to calculate the energy of the input signal over a predetermined duration of calculation (T_{RMS}) during which the amplitude of the input signal does not exceed said peak amplitude threshold (SA).

3. The device of claim 2, further comprising means (32, 42) for inhibiting and reinitializing the means for determining the variable noise level value upon crossing of said peak amplitude threshold before the expiry of said predetermined duration of calculation.

4. The device of claim 1, wherein said peak width threshold (SW) is a fixed predetermined threshold (W₀).

5. The device of claim 1, wherein the digital processing and analysis means further include:
- a low-pass filter (24), adapted to filter said input signal.

6. The device of claim 5, wherein the digital processing and analysis means further include:
- a high-pass filter (20) and a rectifier (22), adapted to filter and rectify the signal applied to the low-pass filter.
